# EUROPEAN PATENT SPECIFICATION

(11) **EP 3 466 409 B1**
(45) Date of publication and mention of the grant of the patent: **07.06.2023**
(21) Application number: 17386032.1
(22) Date of filing: 05.10.2017
(51) Int. Cl.: A61K 9/107, A61K 9/16, A61K 9/20, A61K 9/50, A61K 31/192, A61K 31/519

(54) **SELF-EMULSIFYING COMPOSITIONS OF WEAKLY IONIZABLE OR NON-IONIZABLE ACTIVE PHARMACEUTICAL INGREDIENTS**
SELBSTEMULGIERENDE ZUSAMMENSETZUNGEN VON SCHWACH IONISIERBAREN ODER NICHTIONISIERBAREN, AKTIVEN PHARMAZEUTISCHEN INHALTSSTOFFEN
COMPOSITIONS AUTO-ÉMULSIFIANTES D'INGRÉDIENTS PHARMACEUTIQUES ACTIFS PEU IONISABLES OU FAIBLEMENT IONISABLES

(43) Date of publication of application: 10.04.2019
(73) Proprietor: Vianex S.A., 146 71 Nea Erythrea (GR)
(72) Inventor: Nikolakakis, Ioannis, 55236 Panorama Thessaloniki (GR)
(74) Representative: Roukounas, Dimitrios

(56) References cited:
- US-A1- 2007 026 067
- KRUPA A ET AL: "Preparation of solid self-emulsifying drug delivery systems using magnesium aluminometasilicates and fluid-bed coating process", POWDER TECHNOLOGY, ELSEVIER SEQUOIA, LAUSANNE, CH, vol. 266, 28 June 2014 (2014-06-28), pages 329-339, XP029014492, ISSN: 0032-5910, DOI: 10.1016/J.POWTEC.2014.06.043
- BALAKRISHNAN P ET AL: "Enhanced oral bioavailability of dexibuprofen by a novel solid Self-emulsifying drug delivery system (SEDDS)", EUROPEAN JOURNAL OF PHARMACEUTICS AND BIOPHARMACEUTICS, ELSEVIER SCIENCE PUBLISHERS B.V., AMSTERDAM, NL, vol. 72, no. 3, 1 August 2009 (2009-08-01) , pages 539-545, XP026218291, ISSN: 0939-6411, DOI: 10.1016/J.EJPB.2009.03.001 [retrieved on 2009-03-17]
- MANNINA PAOLO ET AL: "Self-emulsifying excipient platform for improving technological properties of alginate-hydroxypropylcellulose pellets", INTERNATIONAL JOURNAL OF PHARMACEUTICS, ELSEVIER, NL, vol. 499, no. 1, 22 December 2015 (2015-12-22), pages 74-80, XP029405133, ISSN: 0378-5173, DOI: 10.1016/J.IJPHARM.2015.12.048
- SERRATONI ET AL: "Controlled drug release from pellets containing water-insoluble drugs dissolved in a self-emulsifying system", EUROPEAN JOURNAL OF PHARMACEUTICS AND BIOPHARMACEUTICS, ELSEVIER SCIENCE PUBLISHERS B.V., AMSTERDAM, NL, vol. 65, no. 1, 1 December 2006 (2006-12-01), pages 94-98, XP005788729, ISSN: 0939-6411, DOI: 10.1016/J.EJPB.2006.07.011

## Description

### Technical field of the invention

The invention relates to Self-Emulsifying Drug Delivery Systems (SEDDS) of poorly water soluble active pharmaceutical ingredients, pellets comprising these SEDDS and oral pharmaceutical dosage forms comprising these pellets.

### Background of the invention

A great number of class II and class IV drugs according to Biopharmaceutics Classification System (BCS) are formulated into SEDDS, aiming towards solubility and absorption improvement. This is achieved by presenting the drug dissolved in dispersed nanosize lipid droplets, thus, preventing deposition due to low solubility and drug loss. Furthermore, the lipids can increase gastric retention time and for highly lipophilic drugs they may also enhance lymphatic transport and bioavailability by reducing first-pass metabolism (*J*eong SH, Park JH, Park K. In:Wassan KM, editor. Role of lipidexcipients in modifying oral and parenteral drug delivery. New Jersey: Wiley; 2007. p. 32-5).

Medium-chain triglycerides are preferred in the preparation of SEDDS, due to their rapid digestion. Additionally, non-ionic surfactants are preferred in the preparation of SEDDS, because they are less toxic than the ionic, less affected by pH and ionic strength and improve dissolution and absorption of drugs *(*Vervaet C, Baert L, Remon JP. Enhancement of in vitro drug release by using polyethylene glycol 400 and PEG-40 hydrogenated castor oil in pellets made by extrusion/spheronization. Int J Pharm. 1994; 108:207-12*.*
- Swenson ES, Milisen WB, Curatolo W. Intestinal permeability enhancement efficiency: acute local toxicity and reversibility. Pharm Res. 1994; 11:1132-42*.*
- Constantinides PP. Lipid microemulsions for improving drug dissolution and oral absorption: physical and biopharmaceutical aspects. Pharm Res. 1995; 12:1561-72*.*
- Pouton CW, Porter JHC. Formulation of lipid-based delivery systems for oral administration: materials, methods and strategies. Adv Drug Deliv Rev. 2008; 60:625-37).

Numerous articles have been published in the last decade showing improvement of absorption of low solubility drugs incorporated into SEDDS and several pharmaceutical products have appeared in the market. To date, the marketed oral lipid-based formulations are based mostly on semisolid SEDDS lipids filled into capsules, e.g. Neoral^{®} (cyclosporine A) soft gelatin capsules and Solufen^{®} (ibuprofen) hard gelatin capsules *(*Angel Tan & Shasha Rao & Clive A. Transforming Lipid-Based Oral Drug Delivery Systems into Solid Dosage Forms: An Overview of Solid Carriers, Physicochemical Properties, and Biopharmaceutical Performance. Pharm Res. 2013 Dec;30(12):2993-3017*).*

The development of SEDDS into solid dosage forms is a current trend in lipid-based formulation design that besides solubility improvement, has many more advantages over liquid systems. It avoids stringent processing requirements (i), requires less volume of administration (>50% of emulsified SEDDS is water which has been removed from the solid SEDDS particles processed into capsule or tablet) (ii), assists precise dosing (the dose is presented in precise weight of capsule/tablet compared to administration with a syringe or spoon) (iii), offers stability (from chemical degradation and microbial growth) (iv), it is a handy dosage form easy to transfer and store and improves patient compliance (v).

As a multiple-unit dosage form, pellets provide therapeutic advantages of the multiple unit dosage form by assisting smooth passage in the gut thus, reducing the possibility of dose dumping and minimizing variability in plasma levels *(*Newton M, Petterson J, Podczeck F, Clarke A, Booth S. The influence of formulation variables on the properties of pellets containing a self-emulsifying mixture. J Pharm Sci. 2001; 90:987 95*). In vivo* results showed that release of progesterone in dogs from self-emulsifying pellets was equivalent to administration of the microemulsion liquid (Tuleu et al. Comparative Bioavailability Study in Dogs of a Self-Emulsifying Formulation of Progesterone Presented in a Pellet and Liquid Form Compared with an Aqueous Suspension of Progesterone Journal of Pharmaceutical Sciences, Vol. 93, No. 6, June 2004.) and release of dexibuprofen from self-emulsifying spray-dried powder in rats gave significantly higher AUC and Cmax than the dexibuprofen raw powder (P < 0.05). In particular, the AUC of solid SEDDS was about twofold higher than that of dexibuprofen powder (Balakrishnan et al. Enhanced oral bioavailability of dexibuprofen by a novel solid Self-emulsifying drug delivery system (SEDDS). European Journal of Pharmaceutics and Biopharmaceutics 72 (2009) 539-545*)*. US patent application US201213855 discloses the use of SEDDS ingredients and adsorbents to produce free flowing powders and granules and tablets.

Serratoni et al (Controlled drug release from pellets containing water-insoluble drugs dissolved in a self-emulsifying system. European Journal of Pharmaceutics and Biopharmaceutics 65 (2007) 94-98) showed that it is possible to obtain control release of water-insoluble drugs from pellets first by incorporating them into SEDDS to enhance the release rate and then, by applying a polymeric water-insoluble coating containing a water soluble plasticizer and talc, to control the release rate. Changing the coat thickness and/or pre-coating the pellets with a sub-coat of a water-soluble polymer can refine the control of the *in vitro* release of the drug from such pellets to provide a range of release rates.

Hamdani, J. et al. (Physical and thermal characterizations of Precirol and Compritol as lipophilic glycerides used for the preparation of controlled-release matrix pellets. Int. J. Pharm. 2003, 260, 47-57*)* prepared self-emulsifying sustained-release matrix pellets using combinations of glyceryl palmito-stearate (Gelucire 54/02) and glyceryl behenate (Gelucire 70/02).

Patil et al (Effect of formulation variables on preparation and evaluation of gelled self-emulsifying drug delivery system (SEDDS) of ketoprofen. AAPS PharmSciTech September 2004, Volume 5, Issue 3, pp 43-50*)* used colloidal silicon dioxide (CSD) in SEDDS formulations which served the dual purpose of reducing the amount of required solidifying excipients and aiding in slowing down the release of ketoprofen.

Agarwal et al. (Dissolution and powder flow characterization of solid self-emulsified drug delivery system (SEDDS). Int. J. Pharm. 366 (2009) 44-52) investigated the effect of silica-based adsorbents with different chemical nature, specific surface area and particles sizes on the *in vitro* release behaviour of griseofulvin from SEDDS and solid-SEDDS and besides the enhanced dissolution rate of griseofulvin as compared to the micronized drug powder, they also found that SEDDS adsorbed onto Neusilin^{®} UFL2 silicate (5 µm) at 1:1 ratio sustained drug release for longer time.

Zhang et al (Controlled release of cyclosporine A self-nanoemulsifying systemsfrom osmotic pump tablets: Near zero-order release and pharmacokinetics in dogs Int. J. Pharm. International Journal of Pharmaceutics 452 (2013) 233- 240) prepared controlled release tablets of cyclosporine (CyA) by the osmotic release strategy by mixing SEDDS with adsorbents and osmotic tablet core excipients (sucrose, lactose monohydrate, polyethylene oxide, and partly pregelatinized starch) and then transforming into osmotic tablets. Near zero-order release was achieved.

US patent application US2012231083 discloses a method for the preparation of sustained release cannabinoid medicament using SEDDS ingredients together with microcrystalline cellulose and crosscarmellose sodium to form an instant release layer or Methocel K4M to form a slow release layer.

Setthacheewakul et al. (Controlled Release of Oral Tetrahydrocurcumin from a Novel Self-Emulsifying Floating Drug Delivery System (SEFDDS) AAPS PharmSciTech, Vol. 12, No. 1, March 2011*)* used different weight proportions of glyceryl behenate and sodium starch glycolate with SEDDS to obtain pellet formulations with different floating abilities and *in vitro* drug release. The optimum formulation had a floating efficiency of 93% at 6 h and provided a controlled release of Tetrahydrocurcumin over an 8-h period. The pellet formulation was stable under intermediate and accelerated storage conditions for up to 6 months.

Krupa et al. (Preparation of solid self-emulsifying drug delivery systems using magnesium aluminometasilicates and fluid-bed coating process; Powder Technology 266 (2014) 329-339*)* incorporated magnesium aluminometasilicates into pellets in the extrusion/spheronization process, and used such pellets as solid cores for the conversion of liquid SEDDS into solid SEDDS.

The main method that has been used for the preparation of solid-state lipid-based formulations are dry emulsions having SEEDS as the oil phase produced by spray drying or freeze drying or rotary evaporation to remove water. Other more elaborated methods like solid lipid hybrids, microparticles, solid S(M/N)EDDS and proliposomes/proniosomes have also been reported in the literature, but their applicability is limited (*Angel Tan* & *Shasha Rao* & *Clive A. Prestidge D Transforming Lipid-Based Oral Drug Delivery Systems into Solid Dosage Forms: An Overview of Solid Carriers, Physicochemical Properties, and Biopharmaceutical Performance.*

*Pharm Res DOI 10.1007*/*s11095-013-1107-3).* The dry emulsion is essentially fine powder with poor flow characteristics, hampering further processing and manufacturing scale-up. The powder is usually filled into hard gelatin capsules which involves the risk of material sticking to the capsule wall when in contact with the dissolution fluid, thus affecting drug release adversely. Additionally, the dry emulsion powder may be processed into a tablet formulation which however involves the risk of expelling the semisolid oily ingredients during compaction. Furthermore, rotary evaporation and spray drying involve the risk of loss of low melting point, volatile oily constituents, due to heating at high temperatures in the spray drying or application of vacuum in the rotary evaporation process. Additionally, drug migration dissolved in the oily phase to the surface during drying may occur (Nikolakakis et al. Relationships Between the Properties of Self-Emulsifying Pellets and of the Emulsions Used as Massing Liquids for Their Preparation. AAPS PharmSciTech, Vol. 16, No. 1, February 2015*).*

Accordingly, there is a need for pharmaceutical compositions incorporating SEEDS which do not exhibit the disadvantages of the systems known in the art.

### Summary of the invention

The present invention is defined by the claims. The present invention provides SEDDS comprising a mixture of a medium chain triglyceride and a non-ionic surfactant incorporated in solid pellets and produced by extrusion/spheronization.

The pellets of the present invention exhibit the advantages of microemulsions, namely self-emulsification in contact with the stomach fluids, while the active pharmaceutical ingredient is dissolved in the droplets of the lipid carrier, transport towards the gastric mucosa and absorption. At the same time, they exhibit the advantages of pellets, namely lower fluctuation of gastric emptying, narrow distribution of particle sizes, spherical shape and the ability of uniform coating.

The present invention also provides oral tablet pharmaceutical forms comprising the pellets of the present invention.

### Brief description of the drawings

Figure 1 shows comparative plots of the amount of risperidone released from self-emulsifying pellets comprising microcrystalline cellulose (MCC) in comparison to commercially available tablets.
Figure 2 shows the amount of risperidone released in dissolution fluid of pH=1.2 from self-emulsifying pellets prepared with different mixtures of microcrystalline cellulose (MCC)/colloidal silicon dioxide (CSD).
Figure 3 shows the amount of risperidone released in dissolution fluid of pH=6.8 from self-emulsifying pellets prepared with different mixtures of MCC/CSD.
Figure 4 presents plots of the amount of risperidone (%) released from self-emulsifying pellets prepared with a MCC/CSD mixture and coated with a polymeric dispersion.
Figure 5 shows plots of the amount of risperisone released from self-emulsifying pellets comprising a MCC/CSD mixture and tableted with sodium alginate.
Figure 6 shows the friability of ibuprofen pellets containing different amounts of CSD in the MCC/CDS mixture.
Figure 7 shows plots of the amount of ibuprofen (%) release from self-emulsifying pellets prepared with different mixtures of MCC/CSD.

### Detailed description of the invention

According to one aspect, the present invention provides pellets comprising an active pharmaceutical ingredient belonging to the class II or IV according to Biopharmaceutics Classification System, wherein the pellets further comprise a mixture of microcrystalline cellulose (MCC) and colloidal silicon dioxide (CSD) at a weight ratio from 9:1 to 6.5:3.5, wherein the active pharmaceutical ingredient is incorporated into SEDDS comprising a mixture of a medium chain triglyceride and a non-ionic surfactant and wherein the pellets are produced by extrusion/spheronization.

Preferably, the weight ratio of MCC to CSD in the pellets of the present invention is 7.5:2.5 to 6.5:3.5. More preferably, the weight ratio of MCC to CSD is 7:3.

A medium chain triglyceride of the pellets of the present invention is an ester of glycerol with medium chain fatty acids, i.e. with fatty acids having a chain (saturated or unsaturated) of 6 to 12 carbon atoms. Preferably, the medium chain triglyceride is a chemically modified vegetable oil, which in combination with a non-ionic surfactant, easily self-emulsifies in contact with water and its degradation products are similar to those produced during digestion of triglycerides in the human body (T. Gershanik, and S. Benita 2000. Self-dispersing lipid formulations for improving oral absorption of lipophilic drugs Eur. J. Pharm. Biopharm. 50, 179 - 188; V. Jannin et al., 2008. Approaches for the development of solid and semi-solid lipid-based formulations. Adv. Drug Deliver. Rev. 60, 734-746). In addition, a medium chain triglyceride has the advantages of good fluidity, solubilizing ability, drug incorporation ability and self-emulsification ability and, it is less prone to oxidation (Shah et al., 1994. Self-emulsifying drug delivery systems (SEDDS) with polyglycolyzed glycerides for improving in vitro dissolution and oral absorption of lipophilic drugs Int. J. Pharm. 106, 15-23; Kaukonen A. M. et al., 2004. Drug Solubilization Behavior During in Vitro Digestion of Simple Triglyceride Lipid Solution Formulations). More preferably, the medium chain triglyceride is a Caprylic/Capric triglyceride, such as Caprylic/Capric 60/40 triglyceride.

Examples of non-ionic surfactants which may be used in the present invention include polysorbate 20, sorbitan oleate 80, or mixtures thereof. Preferably, the non-ionic surfactant according to the present invention is a mixture of polysorbate 20 and sorbitan oleate at a weight ratio of 9:1.

The active pharmaceutical ingredient of the pellets of the present invention belongs to class II or IV of the Biopharmaceutics Classification System (BCS). According to BCS an active pharmaceutical ingredient belongs to class II when it exhibits high permeability and low solubility, whereas an active pharmaceutical ingredient belongs to class IV when it exhibits low permeability and low solubility. Preferably, the active pharmaceutical ingredient according to the present invention is any weakly ionizable or non-ionizable in acidic environment but non ionizable in pH>4.5 active pharmaceutical ingredient, such as risperidone or ibuprofen. More preferably, the active pharmaceutical ingredient according to the present invention is risperidone.

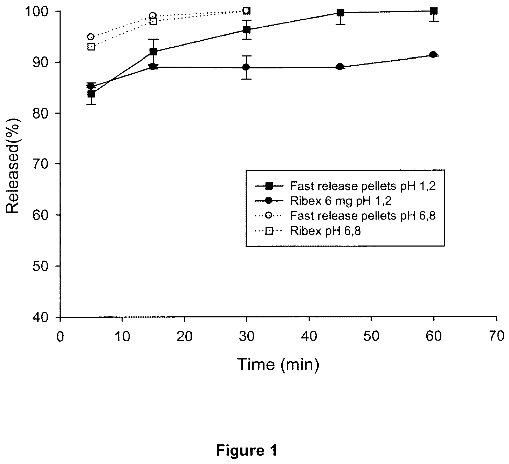

Pellets comprising an active pharmaceutical ingredient incorporated into SEDDS and comprising only microcrystalline cellulose exhibit immediate release. Figure 1 shows comparative plots of the amount of risperidone (%) released from self-emulsifying pellets (loaded with SEDDS), containing only microcrystalline cellulose and prepared by extrusion/spheronization and, the amount (%) released from commercial tablets Ribex^{®} (6mg), in dissolution medium of pH=1.2 (UV analysis at λ=238nm, solid lines) or pH=6.8 (λ=234nm, dotted lines).

Surprisingly, it was found that pellets comprising an active pharmaceutical ingredient belonging to class II or IV of the Biopharmaceutics Classification System incorporated in SEDDS, wherein the pellets comprise a weight ratio of microcrystalline cellulose to colloidal silicon dioxide from 9:1 to 6.5:3.5 and are produced by extrusion spheronisation, exhibit sustained release of the active pharmaceutical ingredient.

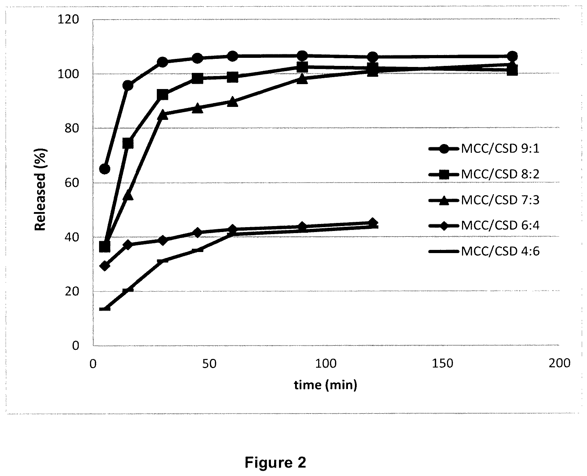

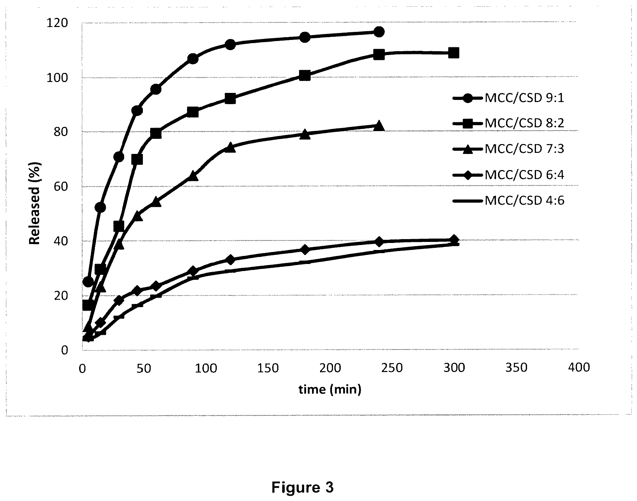

Figure 2 presents plots of risperidone release (%) at pH=1.2 (37 °C, UV analysis at λ=238 nm) from self-emulsifying pellets containing a mixture of MCC/ CSD in weight ratios of 9:1, 8:2, 7:3, 6:4 and 4:6 and prepared by extrusion/spheronization. Similar plots for risperidone release (%) obtained at pH=6.8 (37 °C, UV analysis at λ=234 nm) from self-emulsifying pellets containing a mixture of MCC/CSD in weight ratios of 9:1, 8:2, 7:3, 6:4 and 4:6 and prepared by extrusion/spheronization are presented in Figure 3. At weight ratios of MCC/ CSD of 6:4 and 4:6, the release of the active ingredient reaches a limit after 3 h, with only about 40% of the initial amount of the active ingredient being released.

The pellets of the present invention are produced by extrusion/spheronization, a process well known in the art and extensively studied. For example, the pellets may be produced according to the following process:

A mixture of MCC and CSD is mixed with an emulsion of 40% water and 60% (w/w) self-emulsifying mixture (oil/surfactant/active pharmaceutical ingredient) for about 5 min massing time in a mixing vessel, fitted with a three-blade impeller. Further addition of small amounts of water (decided on the basis of preliminary trials) leads to more spherical pellets. The resulting wet mass is extruded in a radial extruder (Model 20; Caleva Process Solutions, Dorset, UK) operated at 25 rpm and fitted with a 1 mm circular orifice and 1.75 mm thickness screen. The extrudate is immediately processed for 8 min in a spheronizer (Model 120; Caleva Process Solutions, Dorset, UK) fitted with a cross-hatch friction disc. The speed of rotation of the disc is 1360 rpm, determined with a flashing point type digital tachometer (accuracy +9 rpm; Control Ability Ltd., Blackburn, England), and, the corresponding linear perimeter speed of the disc (=2·B·disc radius·1360/60) is 8.55 m/s. The produced wet pellets are dried overnight in an air-circulation tray oven (UT6; Heraeus Instruments, Hanau, Germany) at 40 °C

The pellets of the present invention may be uncoated or they may contain a coating layer. Such coating layer may provide protection from environmental moisture, light and oxygen and sustained release in the gastrointestinal tract. Examples of coating which may be used in the present invention include different acrylic based coatings, Opadry^{®} II coatings, Cellulose Acetate Phthalate coatings, ethylcellulose based and combinations of them like Eudragit^{®} RL, Eudragit^{®} RS, ethyl cellulose/HPMC based coatings Ethylcellulose/PVA-PEG based on the chemical structure, thermal properties and miscibility or solubility of the macromolecules (Siepman et al., Polymer blends for controlled release coatings, J Control Release. 2008 Jan 4;125(1):1-15. Epub 2007 Oct 13*).*

The pellets of the present invention offer a solid dosage form with adjustable release, from more than 90% in 15 min to sustained release of 60% to 80% after 6 h at pH 6.8. They can be produced with conventional equipment and provide a medium for poorly water soluble class II or IV active pharmaceutical ingredients for improvement of their solubility, release rate and absorption within a desirable time dictated by the frequency of administration.

Another aspect of the present invention is an oral pharmaceutical dosage form comprising the pellets of the present invention. Examples of such an oral pharmaceutical dosage form are tablets and capsules. Such forms may be produced by processes well known in the art.

Another aspect of the present invention is a sustained release tablet comprising the pellets of the present invention and a hydrophilic polymer. It has surprisingly been found that such tablets provide well controlled sustained release of the active pharmaceutical ingredient. The hydrophilic polymer should be easily compressible at low compression pressures.

Examples of hydrophilic polymers which may be used in the tablets of the present invention are sodium alginate, hydroxypropylmethyl cellulose, xanthan gum, pectin, chitosan, carrageenan, cellulose ethers and mixtures thereof. Preferably, the hydrophilic polymer is sodium alginate or hydroxypropylmethyl cellulose. More preferably, the hydrophilic polymer is sodium alginate.

The amount of the hydrophilic polymer in the tablets of the present invention is preferably 20% to 40% by weight, depending on the desirable time of prolongation. More preferably, the amount of the hydrophilic polymer which provides the highest prolongation and elimination of burst effect, is 40% by weight.

The use of the pellets together with a hydrophilic polymer in the preparation of tablets comprising the pellets of the present invention provides a dosage form which exhibits sustain release throughout the entire gastrointestinal tract.

### Example 1

This example shows the preparation of pellets according to the present invention containing risperidone as active ingredient.

### Part I. Formulation of Risperidone self-emulsifying oil phase (SEDDS)

The required quantities (% by weight) for the preparation of the SEDDS, are given in Table 1.

**Table 1. Composition of self-emulsifying oil phase (SEDDS)**

| **Component** | **Percentage** |
|---|---|
| Medium chain Caprylic/Capric | 57.6 |
| Polysorbate 20 | 34.8 |
| Sorbitan oleate 80 | 3.8 |
| Risperidone | 3.8 |
| Total | 100 |

For the preparation of Risperidone SEDDS with 4% by weight drug content the following steps were applied:
a) The triglycerides, polysorbate 20 and sorbitan oleate 80 are mixed to give a clear fluid in which risperidone is added and dissolved under light agitation,
b) Deionized water is added to the oil phase under light agitation to give a concentrated oil in water microemulsion of 60% by weight oil phase (SEDDS) with droplet size less than 1 micron and
c) The microemulsion is further subjected to 2 min ultrasonication to smoothen the texture size and reduce the droplet size to around 300-400 nm.

### Part II. Extrusion/Spheronization

The following steps are applied:
a) MCC powder or mixtures of MCC/CSD are added in a Kenwood chef type granulation vessel. In the case of MCC/CSD mixture, due to the different densities, in order to obtain an homogeneous mixture, the two powders are first blended in a Turbula mixer (Bachofen, Switserland) for 10 min.
b) The microemulsion consisting of 60% by weight risperidone oil phase (SEDDS) prepared as described above is added gradually in a time period of about 10 min to the MCC/CDS mixture under stirring using the K-shaped mixing element. The required quantities of MCC/CDS mixture and microemulsion binder are given in Table 2. These ensured consistency of wet paste able to form pellets after extrusion/spheronization. It can be seen that increasing the ratio of CSD in the mixture with MCC, increases the volume of microemulsion required to form pellets and therefore the content of the active ingredient in the final pellets.

**Table 2. Composition of pellet batches comprising only MCC (ratio MCC/CSD 10:0) or a mixture of MCC/CSD at a weight ratio of 8:2 or 7:3 and 4% by weight risperidone in the SEDDS oil phase**

| **MCC/CSD ratio** | **V emulsion (%)#** | **MCC (%)** | **CSD (%)** | **Total** |
|---|---|---|---|---|
| 10:0 | 47.4 | 52.6 | - | 100 |
| 8:2 | 56.5 | 34.8 | 8.7 | 100 |
| 7:3 | 60.8 | 27.4 | 11.8 | 100 |

| | | | | |
|---|---|---|---|---|
| #The density of microemulsion calculated from the individual component densities totals to about 1 so weight (g) and volume (ml) of SEDDS can be used interchangeably | | | | |

c) The wet mass is extruded in a radial type extruder fitted with screen with openings 0.5 or 1 mm, rotating at a rate between 20 to 40 rpm. Screen with small openings of 0.5 mm diameter are recommended for pellets consisting of MCC as the pellet forming material and SEDDS, whereas screen with large openings 1mm or 1.5mm are appropriate for pellets consisting of MCC/CSD mixture as the base of pellets and SEDDS. Other type of extruders like dome-shape can also be used.
d) The extrudate in the form of short spaghetti is fed into the spheronizer with cross hatched protrusions for 5 minutes, rotating at appropriate rotation speed to give peripheral velocity of about 10 m/sec where
# peripheral velocity = 2 * π * disc radius * number of rotations per minute.

This was optimal so as to maintain the cylindrically shaped extruded particles down in contact with the disc of the spheronizer, following at the same time centrifugal movement toward the periphery and developing frictional forces with the wall of the chamber, the surface of the disc and between themselves. Higher speeds made them jump out of the spheronizer chamber whereas smaller speeds kept them down, touching the disc, without centrifugal movement and inadequate friction with the disc surface.

d) The wet pellets were taken out of the spheronizer and spread on trays for drying overnight in an air-circulated tray-oven at 40°C.

### Part III. Coating of self-emulsifying risperidone pellets (SEP)

After their preparation the pellets with risperidone SEDDS are coated to impart protection from environmental factors and improve uptake per os. The protective polymeric coating applied is Opadry^{®} II based on polyvinyl alcohol and its application follows the instructions of the manufacturer.

Additionally, application of appropriate polymeric coating renders sustained release of the drug from the coated pellets. As already mentioned, the dissolution tests on uncoated pellets showed that the presence of CSD delays considerably the release of drug in dissolution medium of pH 6.8 where risperidone is poorly soluble, but there is no such delay in dissolution medium of pH 1.2 where the drug solubility is higher. Therefore to delay the release at pH 1.2 and enable sustained release of drug throughout the gastrointestinal tract (stomach and intestine) a combination of methacrylic type polymers: Eudragit^{®} L30 D55 enteric which is insoluble and suppresses release in pH 1.2 combined with Eudragit^{®} NE 30 D (Evonic Industries, Germany) which has low water permeability independent of pH in ratio 1:1 is used.

### Composition and preparation of the polymeric coating dispersion

| | |
|---|---|
| 50 g | L30 D55 |
| 5 g | 1N NaOH |
| 50 g | NE 30D |
| 15 g | talc |
| 120 g | H2O deionised |

The polymeric dispersion of L30 D55 is stirred for 15 min and NaOH is slowly added under continuous stirring for another 15 min. Then, the dispersion of NE 30D is added slowly and stirred for further 15 min. Finally, in the mixture of the two polymeric dispersions talc fine powder is added and homogenized using UltraTurax homogenizer for 15 min.

### Settings on the coater/drier:

| | |
|---|---|
| Fan | 1% |
| Agitation | 23% |
| Temperature | 35 oC |
| Pump | 90% |
| Time for coating | 5% 8 min |
| Time for coating | 10% 13 min. |

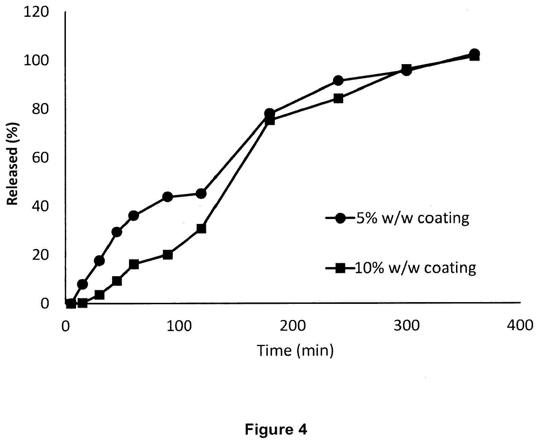

Figure 4 shows the plots of the amount of risperidone (%) released at pH=1.2 for the first 120 min and at ρH=6.8 for the rest 240 min, from pellets coated either with 5% by weight (polymeric dispersion : pellet core) or with 10% by weight (polymeric dispersion : pellet core). The results show sustained release of the active ingredient.

### Example 2

This example shows the preparation and release characteristics of sustained release risperidone tablets comprising uncoated pellets, prepared according to Part I and Part II of Example 1.

Risperidone self-emulsifying pellets of about 2% by weight risperidone content were compressed into tablets containing 6 mg risperidone, using sodium alginate at 20 or 40% proportion of tablet weight as a tableting (and sustained release at pH 1.2) aid. An automatic single punch tableting machine fitted with 11-mm flat face punches was used (Korsch-Erweka, Germany) and relatively low compression pressures between 70 to 90 psi were applied.

**Table 3. Composition (mg) of tablet of self-emulsifying risperidone pellets (MCC/CSD weight ratio 7:3 and 2% by weight drug) using sodium alginate (0%, 20%, 30% and 40% by weight) as tableting aid#**

| **Batch** | **Pellets (mg)** | **Sodium alginate (mg)** | **Weight of tablet (mg)** |
|---|---|---|---|
| T1 | 300 | --- | 300 |
| T2 | 300 | 75 | 375 |
| T3 | 300 | 130 | 430 |
| T4 | 300 | 200 | 500 |

| | | | |
|---|---|---|---|
| # Compression pressure 70 bar, drug content 2% w/w | | | |

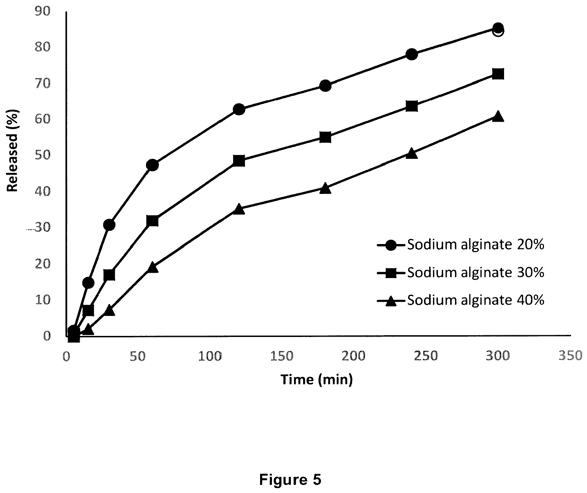

From the drug release profiles shown in Figure 5 it can be see that tablets of self - emulsifying risperidone pellets after 5 h testing time, they release 50%, 65% and 80% risperidone for 20%, 30% and 40% sodium alginate content respectively.

### Example 3

This example shows the preparation of pellets according to the present invention containing ibuprofen base as the active ingredient.

### Part I. Formulation of Ibuprofen self-emulsifying oil phase (SEDDS)

The required quantities (% by weight) for the preparation of the SEDDS, are given in Table 4.

**Table 4. Composition of ibuprofen self-emulsifying oil phase (SEDDS)**

| **Component** | **Percentage** |
|---|---|
| Medium chain Caprylic/Capric triglycerides (60/40) | 37.5 |
| Polyoxyl 35 Castor Oil oil (Cremophor^{®} EL) | 25.0 |
| Ibuprofen | 37.5 |
| Total | 100 |

For the preparation of ibuprofen SEDDS with 37.5% by weight drug content the following steps were applied:
a) The triglycerides and the surfactant Polyoxyl 35 Castor Oil, are mixed to give a clear fluid in which Ibuprofen is added and dissolved under light agitation
b) Deionized water is added to the oil phase under light agitation to give a concentrated oil in water microemulsion of 60% by weight oil phase (SEDDS) with droplet size less than 1 micrometer and
c) The microemulsion is further subjected to 2 min ultrasonication to smoothen the texture size and reduce the droplet size to around 300-400 nm.

### Part II. Extrusion/Spheronization

The following steps are applied:
a) MCC powder only or mixtures of MCC/CSD are added in a Kenwood chef type granulation vessel. In the case of MCC/CSD mixture, due to the different densities, in order to obtain an homogeneous mixture, the two powders are first blended in a Turbula mixer (Bachofen, Switserland) for 10 min.
b) The microemulsion consisting of 60% by weight ibuprofen oil phase (SEDDS) prepared as described above is added gradually in a time period of about 10 min to the MCC/CSD mixture under stirring using the K-shaped mixing element. The required quantities of MCC/CSD mixutre and microemulsion (binder) are given in Table 5. These ensured consistency of wet paste able to form pellets after extrusion/spheronization. It can be seen that increasing the ratio of CSD in the mixture with MCC, increases the volume of microemulsion required to form pellets and therefore the content of the active ingredient in the final pellets.

**Table 5. Composition of pellet batches comprising only MCC (weight ratio MCC/CSD 10:0) or a mixture of MCC/CSD at a weight ratio of 7:3 or 8:2 and 37.5% by weight ibuprofen in the SEDDS oil phase**

| **MCC/CSD ratio** | **V emulsion (%)** | **MCC (%)** | **CSD (%)** | **Total** |
|---|---|---|---|---|
| 10:0 | 46.0 | 54.0 | 0 | 100 |
| 7:3 | 43.8 | 16.9 | 39.3 | 100 |
| 6:4 | 58.0 | 25.2 | 16.8 | 100 |

c) The wet mass is extruded in a radial type extruder fitted with screen with openings 0.5 or 1 mm, rotating at a rate between 20 to 40 rpm. Screen with small openings of 0.5 mm diameter are recommended for pellets consisting of MCC as the pellet forming material and SEDDS, whereas screen with large openings 1mm or 1.5mm are appropriate for pellets consisting of MCC/CSD mixture as the base of pellets and SEDDS. Other type of extruders like dome-shape can also be used.
d) The extrudate in the form of short spaghetti is fed into the spheronizer with cross hatched protrusions for 5 minutes, rotating at appropriate rotation speed to give peripheral velocity of about 10 m/sec where
peripheral velocity = 2 * π * disc radius * number of rotations per minute.

This was optimal so as to maintain the cylindrically shaped extruded particles down in contact with the disc of the spheronizer, following at the same time centrifugal movement toward the periphery and developing frictional forces with the wall of the chamber, the surface of the disc and between themselves. Higher speeds made them jump out of the spheronizer chamber whereas smaller speeds kept them down, touching the disc, without centrifugal movement and inadequate friction with the disc surface.

d) The wet pellets were taken out of the spheronizer and spread on trays for drying overnight in an air-circulated tray-oven at 40°C.

From Table 5 it can be seen that consumption of emulsion and therefore drug content in the pellets after drying increase considerably with increasing amount of CSD in the MCC/CSD powder mixture.

From Table 6 it can be seen that the pellets with higher CSD content also have greater diameter. The higher diameter is due to the greater emulsion consumption and therefore greater total pellet mass.

**Table 6. Mean diameter and aspect ratio (optical microscopy) of pellet batches prepared with MCC/CSD mixture in weight ratios 10:0, 7:3 and 4:6 and 37.5% by weight ibuprofen in the SEDDS oil phase**

| **MCC/CSD** | **Median diameter (µm)** | **Aspect ratio** |
|---|---|---|
| 10:0 | 1070 | 1.10 |
| 7:3 | 1300 | 1.10 |
| 4:6 | 1250 | 1.13 |

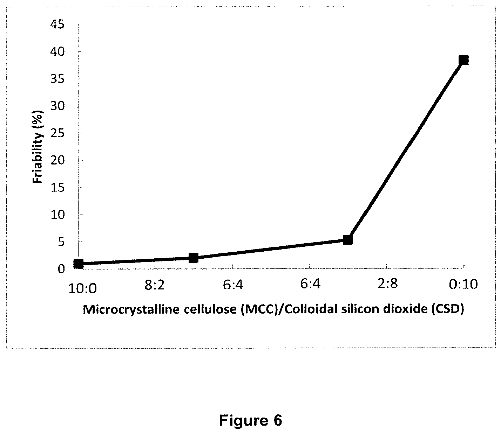

The shape of the pellets containing only MCC or a mixture of MCC/CSD at a weight ratio of 7:3 are spherical with relatively smooth surface, whereas those containing a mixture of MCC/CSD at a weight ratio of 4:6 although spherical, have rough surface. From the friability vs ratio MCC/CSD plots in Figure 6, it is clear that pellets with a MCC/CSD weight ratio higher (with respect to the CSD amount) than 7.5:2.5 have very high friability which makes them inappropriate for further processing.

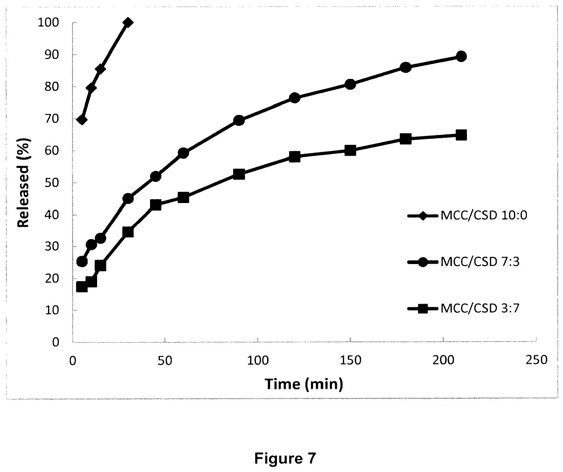

Figure 7 shows the release profile of ibuprofen. It can be seen that pellets comprising ibuprofen incorporated in SEDDS wherein the pellets produced by extrusion/ spheronization comprise a weight ratio of MCC/CSD of 9:1 7:3 and 4:6, exhibit sustained release of the active pharmaceutical ingredient, which is true for both pH 1.2 or pH 6.8 of dissolution fluid (curves in Figure 10 are from tests at pH 6.8 but no significant difference is expected at pH 1.2). Drug release is progressing over the 3 h studied, and retardation increases as the CSD weight percentage in the CSD/MCC mixture increases from 0% up to 30%. At the higher CSD% 60% studied, release reaches a limit after the 3 h of test with only about 60% of the initial amount of the active ingredient in the formulation released.

## Claims

1. Pellets comprising an active pharmaceutical ingredient belonging to the class II or IV according to Biopharmaceutics Classification System, wherein the pellets further comprise a mixture of microcrystalline cellulose and colloidal silicon dioxide at a weight ratio from 9:1 to 6.5:3.5, wherein the active pharmaceutical ingredient is incorporated in SEDDS comprising a mixture of a medium chain triglyceride and a non-ionic surfactant and wherein the pellets are produced by extrusion/spheronization.

2. Pellets according to claim 1, wherein the weight ratio of microcrystalline cellulose to colloidal silicon dioxide is from 7.5:2.5 to 6.5:3.5.

3. Pellets according to claim 2, wherein the weight ratio of microcrystalline cellulose to colloidal silicon dioxide is 7:3.

4. Pellets according to any one of claims 1 to 3, wherein the medium chain triglyceride is a Caprylic/Capric triglyceride.

5. Pellets according to any one of claims 1 to 4, wherein the surfactant is selected from the group consisting of polysorbate 20, sorbitan oleate 80 and mixtures thereof.

6. Pellets according to any one of claims 1 to 5, wherein the surfactant is a mixture of polysorbate 20 and sorbitan oleate at a weight ratio of 9:1.

7. Pellets according to any one of claims 1 to 6, wherein the active pharmaceutical ingredient is selected from the group consisting of risperidone and ibuprofen.

8. Pellets according to any one of claims 1 to 7, wherein the active pharmaceutical ingredient is risperidone.

9. An oral pharmaceutical dosage form comprising the pellets of any one of claims 1 to 8.

10. An oral pharmaceutical dosage form according to claim 9, wherein the dosage form is a tablet.

11. A sustained release tablet comprising the pellets of any one of claims 1 to 8 and a hydrophilic polymer.

12. A sustained release tablet according to claim 11, wherein the hydrophilic polymer is selected from the group consisting of sodium alginate, hydroxypropylmethyl cellulose, xanthan gum, pectin, chitosan, carrageenan, cellulose ethers and mixtures thereof.

13. A sustained release tablet according to claim 11 or 12, wherein the hydrophilic polymer is sodium alginate.

14. A sustained release tablet according to any one of claims 11 to 13, wherein the amount of the hydrophilic polymer in the tablet is from 20% to 40% by weight.

15. A sustained release tablet according to any one of claims 11 to 14, wherein the amount of the hydrophilic polymer in the tablet is 40% by weight.

## Patentansprüche

1. Pellets, umfassend einen pharmazeutischen Wirkstoff, der gemäß Biopharmazeutischem Klassifizierungssystem zur Klasse II oder IV gehört, wobei die Pellets ferner eine Mischung aus mikrokristalliner Cellulose und kolloidalem Siliziumdioxid in einem Massenverhältnis von 9:1 bis 6,5:3,5 umfassen, wobei der pharmazeutische Wirkstoff in SEDDS, umfassend eine Mischung aus einem mittelkettigen Triglycerid und einem nichtionischen Tensid, integriert ist, und wobei die Pellets durch Extrusion/Sphäronisation hergestellt werden.

2. Pellets nach Anspruch 1, wobei das Massenverhältnis von mikrokristalliner Cellulose zu kolloidalem Siliziumdioxid von 7,5:2,5 bis 6,5:3,5 beträgt.

3. Pellets nach Anspruch 2, wobei das Massenverhältnis von mikrokristalliner Cellulose zu kolloidalem Siliziumdioxid 7:3 beträgt.

4. Pellets nach einem der Ansprüche 1 bis 3, wobei das mittelkettige Triglycerid ein Capryl-/Caprin-Triglycerid ist.

5. Pellets nach einem der Ansprüche 1 bis 4, wobei das Tensid aus der Gruppe, die aus Polysorbat 20, Sorbitanoleat 80 und Mischungen davon besteht, ausgewählt ist.

6. Pellets nach einem der Ansprüche 1 bis 5, wobei das Tensid eine Mischung aus Polysorbat 20 und Sorbitanoleat in einem Massenverhältnis von 9:1 ist.

7. Pellets nach einem der Ansprüche 1 bis 6, wobei der pharmazeutische Wirkstoff aus der Gruppe, die aus Risperidon und Ibuprofen besteht, ausgewählt ist.

8. Pellets nach einem der Ansprüche 1 bis 7, wobei der pharmazeutische Wirkstoff Risperidon ist.

9. Orale pharmazeutische Darreichungsform, umfassend die Pellets nach einem der Ansprüche 1 bis 8.

10. Orale pharmazeutische Darreichungsform nach Anspruch 9, wobei die Darreichungsform eine Tablette ist.

11. Tablette mit verzögerter Freisetzung, umfassend die Pellets nach einem der Ansprüche 1 bis 8 und ein hydrophiles Polymer.

12. Tablette mit verzögerter Freisetzung nach Anspruch 11, wobei das hydrophile Polymer aus der Gruppe, die aus Natriumalginat, Hydroxypropylmethylcellulose, Xanthangummi, Pektin, Chitosan, Carrageen, Celluloseethern und Mischungen davon besteht, ausgewählt ist.

13. Tablette mit verzögerter Freisetzung nach Anspruch 11 oder 12, wobei das hydrophile Polymer Natriumalginat ist.

14. Tablette mit verzögerter Freisetzung nach einem der Ansprüche 11 bis 13, wobei die Menge des hydrophilen Polymers in der Tablette einen Massenanteil von 20 % bis 40 % ausmacht.

15. Tablette mit verzögerter Freisetzung nach einem der Ansprüche 11 bis 14, wobei die Menge des hydrophilen Polymers in der Tablette einen Massenanteil von 40 % ausmacht.

## Revendications

1. Billes comprenant un principe actif appartenant à la Classe II ou à la Classe IV selon le Système de classification biopharmaceutique, où les billes comprennent en outre un mélange de cellulose microcristalline et de dioxyde de silicone colloïdal dans un rapport de poids de 9:1 à 6,5:3,5, où le principe actif est incorporé dans des systèmes d'administration de médicaments auto-émulsifiants (SEDDS) comprenant un mélange d'un triglycéride à chaîne moyenne et un tensioactif non ionique, et où les billes sont produites par extrusion/sphéronisation.

2. Billes selon la revendication 1, où le rapport de poids entre la cellulose microcristalline et le dioxyde de silicone colloïdal est de 7,5:2,5 à 6,5:3,5.

3. Billes selon la revendication 2, où le rapport de poids entre la cellulose microcristalline et le dioxyde de silicone colloïdal est de 7:3.

4. Billes selon l'une quelconque des revendications 1 à 3, où le triglycéride à chaîne moyenne est un triglycéride caprylique/caprique.

5. Billes selon l'une quelconque des revendications 1 à 4, où le tensioactif est sélectionné dans le groupe comprenant le polysorbate 20, l'oléate de sorbitane 80 ou des mélanges de ceux-ci.

6. Billes selon l'une quelconque des revendications 1 à 5, où le tensioactif est un mélange de polysorbate 20 et d'oléate de sorbitane dans un rapport de poids de 9:1.

7. Billes selon l'une quelconque des revendications 1 à 6, où le principe actif est sélectionné dans le groupe comprenant la rispéridone et l'ibuprofène.

8. Billes selon l'une quelconque des revendications 1 à 7, où le principe actif est la rispéridone.

9. Formulation pharmaceutique par voie orale comprenant les billes selon l'une quelconque des revendications 1 à 8.

10. Formulation pharmaceutique par voie orale selon la revendication 9, où la formulation est un comprimé.

11. Comprimé à libération prolongée comprenant les billes selon l'une quelconque des revendications 1 à 8, ainsi qu'un polymère hydrophile.

12. Comprimé à libération prolongée selon la revendication 11, où le polymère hydrophile est sélectionné dans le groupe comprenant l'alginate de sodium, l'hydroxypropylméthylcellulose, la gomme de xanthane, la pectine, le chitosan, les carraghénanes, les éthers de cellulose et les mélanges de ceux-ci.

13. Comprimé à libération prolongée selon les revendications 11 ou 12, où le polymère hydrophile est l'alginate de sodium.

14. Comprimé à libération prolongée selon l'une quelconque des revendications 11 à 13, où la quantité du polymère hydrophile dans le comprimé représente 20 % à 40 % du poids.

15. Comprimé à libération prolongée selon l'une quelconque des revendications 11 à 14, où la quantité du polymère hydrophile dans le comprimé représente 40 % du poids.
